# EUROPÄISCHE PATENTANMELDUNG

(11) **EP 2 277 547 A1**
(43) Veröffentlichungstag der Anmeldung: **26.01.2011**
(21) Anmeldenummer: 09009393.1
(22) Anmeldetag: 20.07.2009
(51) Int. Cl.: A61K 47/48, A61K 49/08, A61K 51/04

(54) **Epsilon-Polylysin-Konjugate und deren Verwendung**

(71) Anmelder: Merck Patent GmbH, 64293 Darmstadt (DE)
(72) Erfinder: Larbig, Gregor Dr., 63571 Geinhausen (DE); Kübelbeck, Armin, 64625 Bensheim (DE); Mier, Walter Dr., 64625 Bensheim (DE); Beijer, Barbo Dr., 69226 Nussloch (DE); Haberkorn, Uwe, Dr., Prof., 68723 Schwetzingen (DE)

(57) **Zusammenfassung**

Die vorliegende Erfindung betrifft ε-Polylysin-Konjugate, insbesondere Konjugate von ε-Polylysin mit Carboxylgruppen tragenden Verbindungen, sowie deren Herstellung und Verwendung für das Targeting der Niere.

## Beschreibung

Die vorliegende Erfindung betrifft ε(epsilon)-Polylysin-Konjugate, insbesondere Konjugate von ε-Polylysin mit Carboxylgruppen tragenden Verbindungen, sowie deren Herstellung und Verwendung für das Targeting der Niere.

Die Niere ist ein Organ, das insbesondere für den Transport und die Ausscheidung verschiedener Substanzen und bei der Produktion von Hormonen von Bedeutung ist.
Eine Funktion der Nieren ist die Ausscheidung von Endprodukten des Stoffwechsels, den sogenannten harnpflichtigen Substanzen, und Giftstoffen aus dem Körper durch Bildung des Harns, welcher schließlich über die Harnwege aus dem Körper ausgeschieden wird. Die Niere bilanziert den Wasserhaushalt und dient damit der langfristigen Blutdruckeinstellung. Sie reguliert durch die Kontrolle der Zusammensetzung des Harns den Elektrolythaushalt und den Säure-Basen-Haushalt. Weiterhin ist die Niere ein bedeutendes Organ für den Zwischenstoffwechsel des Körpers (sie betreibt Gluconeogenese). Die Niere produziert Hormone, wie beispielsweise Erythropoetin, für die Blutbildung und ist der Abbauort von Peptidhormonen. Aber auch viele Funktionen der Niere selbst werden durch Hormone gesteuert.

Daher ist die Niere ein lebenswichtiges Organ, für das bereits viele diagnostische und therapeutische Methoden entwickelt wurden. Beispielsweise werden zur Behandlung der Niere bzw. zur Beeinflussung der Nierenfunktion Immunsuppressiva, Zytostatika, Immuntherapeutika, Antiphlogistika, Antibiotika, Virostatika, Antihypertensiva, Urikosurika, oder Diuretika eingesetzt. Besonders wichtig ist es dabei, dass die Medikamente so gezielt wie möglich in die Niere gelangen.

Genauso ist auch die Darstellung der Niere in bildgebenden Verfahren von großer Bedeutung.

Mit Hilfe der etablierten nuklearmedizinischen und radiologischen Verfahren wie SPECT, PET, Ultraschall und MRT können neben morphologischen Strukturen durch die sogenannte molekulare Bildgebung enzymatische Prozesse, Stoffwechselvorgänge, die Expression bestimmter Gene und molekulare Reaktionen dargestellt werden. Die oben genannten Bildgebungsmodalitäten können eventuell weiter durch computertomographische und optische Bildgebungsverfahren (Near-Infrared-Imaging) ergänzt werden. Der Schwerpunkt des "Molecular Imaging" liegt derzeit noch in der Diagnostik von Krebserkrankungen, neurologischen Fragestellungen und der Kontrolle von Gentherapien, wird jedoch in der Zukunft auf alle Bereiche, in denen zelluläre Veränderungen möglichst frühzeitig entdeckt werden müssen, erweitert werden.

Als Signalquelle wird für die bildgebenden Verfahren in der Regel ein "Signalmolekül" mit einem "Trägermolekül" gekoppelt. Das "Trägermolekül" sorgt für das möglichst gezielte Targeting, indem es z.B. spezifisch an die Zielzellen bindet oder in diesen getrappt wird. Beispielsweise kann es sich bei dem Trägermolekül um den Liganden eines Rezeptors oder um das Substrat eines Enzyms handeln. Das "Signalmolekül" kann mittels einer oder mehrerer Bildgebungstechniken sichtbar gemacht werden. Beispiele für Signalmoleküle sind z.B. Komplexbildner oder Chelatoren, deren Metallionen über Bildgebungstechniken detektiert werden können. Die Verbindung bzw. das Konjugat aus Signalmolekül und Trägermolekül wird "Diagnostikum" genannt. Im Folgenden sollen die verschiedenen Bildgebungstechniken im Detail diskutiert werden.

### Computer-Tomographie (CT)

Bei der klassischen Röntgenuntersuchung wird die gewebespezifische Abschwächung von Röntgenstrahlen auf einem Röntgenfilm dargestellt. Hierbei absorbieren "harte Gewebe", z.B. Knochen im Gegensatz zu "weichem" Gewebe wie Fett und Muskeln viel Strahlung. Als Röntgen-Kontrastmittel dienen Substanzen, die Elemente mit einer hohen Ordnungszahl enthalten, beispielsweise lod-haltige Moleküle für die Angiographie. Als Weiterentwicklung liefert die Röntgenuntersuchung Querschnittsbilder. Bei der CT werden Röntgenaufnahmen aus verschiedenen Richtungen von Sensoren (Detektoren), aufgezeichnet und per Computer als dreidimensionale Röntgenuntersuchung wiedergegeben. Aufgrund der breiten Anwendbarkeit der CT wird dieses Verfahren als das "Arbeitspferd der klassischen Radiologie" bezeichnet. Die geringe Empfindlichkeit des Verfahrens begrenzt aber seinen Einsatz als Methode zur molekularen Bildgebung.

### Single-Photon-Emissionscomputertomographie (SPECT)

Die Szintigraphie nutzt die von kurzlebigen Radionukliden ausgesendete Gamma-Strahlung radioaktiv markierter Stoffe (Radiotracer). Im Körper reichern sich diese spezifisch im Zielgewebe an. Mit Hilfe einer Gammakamera wird die ausgesandte Strahlung erfasst und in ein Bild umgewandelt. Die *Single Photon Emission Computed Tomography* (SPECT) ist die dreidimensionale Variante der Szintigraphie. Bei der SPECT wird die Strahlung so wie bei der CT aus verschiedenen Winkeln registriert, und im Computer wird ein dreidimensionales Bild erhalten. Bei einem statischen SPECT wird die Konzentration des Radiotracers zu einem bestimmten Zeitpunkt gemessen. Bei der dynamischen SPECT wird die Messung in bestimmten Zeitabständen wiederholt. Auf diese Weise kann der Verlauf der Anreicherung untersucht werden.

### Positronen-Emissions-Tomographie (PET)

In der klinischen Anwendung ergänzt die PET die stärker strukturell orientierten bildgebenden Verfahren der diagnostischen Radiologie. Die Positronen-Emissions-Tomographie (PET) ist ein modernes funktionell bildgebendes Verfahren. Es ermöglicht mit Hilfe von Positronen - emittierenden Atomen eine hervorragende Auflösung bei der Detektion von Radioisotopen (in modernen PET-Geräten wird selbst bei Ganzkörpertomographen eine Auflösung von 2-3 mm erreicht). Werden diese Radioisotope, z.B. ⁶⁸Ga, zur Markierung von Biomolekülen eingesetzt, so gelingt es, die biochemischen Vorgänge des Organismus in einzelnen Organen abzubilden. Ein wesentlicher Vorteil der nuklearmedizinischen Verfahren ist die hohe Empfindlichkeit, aufgrund der die Tracer nur winzigsten Mengen (Nanogrammmengen) eingesetzt werden können. Heute wird die PET-Kamera mit einem CT-Gerät (dieses bietet eine hohe Ortsauflösung von ca. < 1 mm) fusioniert. Die PET/CT-Technologie hat in den vergangenen Jahren revolutionäre Neuigkeiten in die Diagnostik eingebracht. Somit können die funktionellen Bilder der PET und die morphologischen Informationen der CT in gleichen anatomischen Strukturen in einer Darstellung erhalten werden.

Ein in der medizinischen Diagnostik sehr häufig und sehr intensiv untersuchtes Organ ist die Niere. Häufigste Untersuchungsmethode hierbei ist die Nierenszintigraphie.

### Nierenszintigraphie

Die Nierenszintigraphie ist ein nuklearmedizinisches
Untersuchungsverfahren, welches die Beurteilung der Nierenfunktion unter statischen und dynamischen Gesichtspunkten erlaubt. Beurteilt werden dabei die Blutversorgung, Funktion und Exkretion jeder einzelnen Niere. Es ist ein etabliertes Verfahren zur Erkennung von Parenchymnarben, insbesondere bei Kindern, und dient weiter zur Beurteilung der regionalen und seitengetrennten Nierenfunktion.
Es wird zwischen zwei Formen der Nierenszintigraphie unterschieden:

### Statische Nierenszintigraphie

Bei der statischen Nierenszintigraphie wird unter Verwendung des Radionuklids ^{99m}Tc das funktionsfähige Nierengewebe dargestellt. Das Technetium ist dabei beispielsweise an 2,3-Dimercaptosuccinsäure (DMSA) komplex gebunden. Die statische Nierenszintigraphie eignet sich daher vor allem zur Darstellung von Nieren mit Anomalien (Dystrophie, Hufeisenniere etc.) oder Zustand nach Entzündung.

### Dynamische Nierenszintigraphie

Die dynamische Nierenszintigraphie untersucht demgegenüber die Nierenfunktion. So können die glomeruläre Filtrationsrate, der renale Blutfluss (RBF="renal blood flow") und die tubuläre Sekretion mit der Fragestellung nach der Nierenfunktion und ihrer Clearance untersucht werden.

Als Radiopharmaka kommen gegenwärtig die folgenden Substanzen zum Einsatz:
- ^{99m}Tc-MAG3 Mercaptoacetyl-triglycin
- ^{99m}Tc-DMSA 2,3-Dimercaptosuccinsäure
- ^{99m}Tc-DTPA Diethylentriaminpentaessigsäure
- ¹²³I-OIH Hippuran (Orthoiodhippursäure)

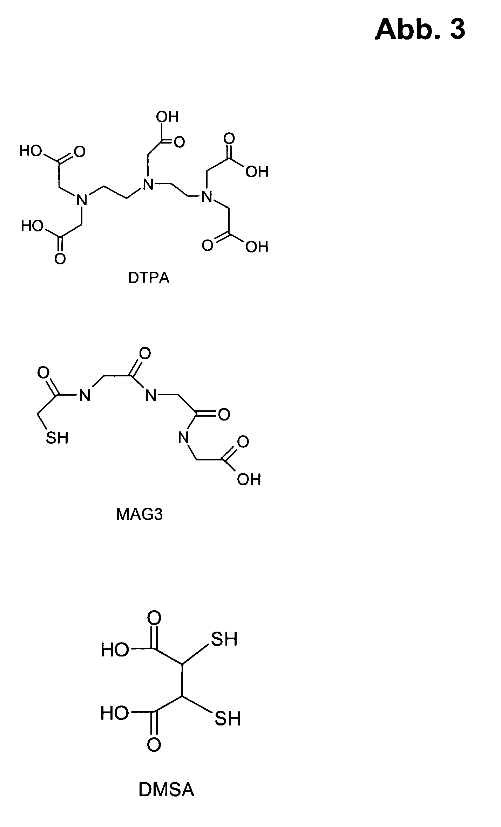

Abbildung 3 zeigt die chemische Struktur von MAG3, DMSA und DTPA.

Eine Nierenfunktionsszintigraphie (= dynamische Nierenszintigraphie) wird bei den folgenden Indikationen eingesetzt:
- Zur Abklärung der seitengetrennten Nierenfunktion bei Nierenerkrankungen, wie beispielsweise Nierensteinen (Nephrolithiasis), Nierentumoren, dystrophen (am falschen Ort befindlichen) oder dysplastischen (fehlgebildeten) Nieren
- zur Untersuchung der Teilfunktion bei Doppelnieren
- zur Untersuchung von Harnabflussstörungen
- zur Abklärung eines vesikorenalen Refluxes (eine Anomalie der harnableitenden Wege)
- bei Verdacht einer renovaskulären Hypertonie
- zur Nierenfunktionsprüfung vor einer Nierenlebendspende
- zur Verlaufskontrolle operativ versorgter Gefäßverengungen oder - verschlüsse (Obstruktionen)
- zur Beurteilung von transplantierten Nieren
- in der Notfalldiagnostik bei Verdacht auf eine Verletzung der Nieren (Nierentrauma)
- bei plötzlich auftretender stark reduzierter Harnausscheidung (Anurie) zum Ausschluss einer Nierenembolie oder eines akuten
   Harnablaufstaus
- zur Bestimmung der Gesamt-Clearance
- zum Nachweis, beziehungsweise zum Ausschluss, einer Urinleckage

Bislang erfolgt die Nierenfunktionsszintigraphie in der Regel mittels SPECT, da die zugelassenen Tracer MAG3 und DMSA nur mit dem SPECT-Nuklid ^{99m}Tc markiert werden können. Folglich können die heute durch die PET und PET/CT erheblich verbesserten Möglichkeiten bislang nicht für die Nierenfunktionsdiagnostik genutzt werden.

Auch für therapeutische Zwecke wäre es wünschenswert, wenn das Targeting der Niere verbessert werden könnte.

Aufgabe der vorliegenden Erfindung war es daher, einen Carrier bzw. ein Trägermolekül für ein Therapeutikum oder ein Diagnostikum zur Verfügung zu stellen, das eine möglichst hohe Affinität und Selektivität für die Niere aufweist.

Es wurde überraschend gefunden, dass Konjugate von ε-Polylysin oder ε-Polylysin-Derivaten mit Carboxylgruppen tragenden Verbindungen eine extrem hohe Selektivität für die Niere aufweisen. Das bedeutet, diese Konjugate werden fast ausschließlich vom Nierengewebe aufgenommen. Diese Konjugate können mit Signalmolekülen wie z.B. Radioisotopen und/oder Wirkstoffen gekoppelt für die diagnostische und/oder therapeutische Behandlung der Niere eingesetzt werden.

Gegenstand der vorliegenden Erfindung ist daher ein Konjugat enthaltend zumindest eine Carboxylgruppen tragende Verbindung und ein lineares oder verzweigtes Oligomer, das aus peptidisch verknüpften Monomereinheiten besteht und das entweder insgesamt zu mehr als 50% (bezogen auf die Anzahl der Monomereinheiten) aus ε-Lysin-Monomereinheiten aufgebaut ist oder mindestens 10 aufeinander folgende Monomereinheiten enthält, die mindestens zu 70% (bezogen auf die Anzahl der Monomereinheiten) aus ε-Lysin-Monomereinheiten aufgebaut sind.

In einer bevorzugten Ausführungsform für therapeutische Anwendungen enthält das Konjugat zusätzlich zumindest einen typischerweise kovalent gebundenen Wirkstoff.

In einer bevorzugten Ausführungsform weist das Oligomer eine Kettenlänge von 10 bis 50 Monomereinheiten auf.

In einer besonders bevorzugten Ausführungsform besteht das Oligomer nur aus ε-Lysin-Monomereinheiten, insbesondere nur aus ε-Lysin-Einheiten.

In einer bevorzugten Ausführungsform ist zumindest eine Carboxylgruppen tragende Verbindung über die Aminogruppe einer ε-Lysin-Monomereinheit gebunden, d.h. ein oder mehrere ε-Lysin-Monomereinheiten tragen an ihrer Aminogruppe eine direkt oder über einen Spacer konjugierte Carboxylgruppen tragende Verbindung.

In einer Ausführungsform, die besonders für diagnostische Anwendungen bevorzugt ist, handelt es sich bei der Carboxylgruppen tragenden Verbindung um einen Komplexbildner, besonders bevorzugt um DOTA (= 1,4,7,10-Tetraaza-cyclododecan-N, -N', -N", -N"'-Tetraessigsäure) oder DTPA (Diethylentriaminpentaessigsäure).

Gegenstand der vorliegenden Erfindung ist auch ein Verfahren zur Herstellung der erfindungsgemäßen Verbindungen, das zumindest die folgenden Verfahrensschritte aufweist:
a) Bereitstellen eines erfindungsgemäßen Oligomers, das zumindest eine reaktive Gruppe aufweist,
b) Konjugation mindestens einer optional aktivierten Carboxylgruppen tragenden Verbindung an das Oligomer aus Schritt a)

In einer Ausführungsform des erfindungsgemäßen Verfahrens, falls das Konjugat einen Komplexbildner enthält, wird in einem weiteren Schritt c) die in Schritt b) erhaltene Verbindung mit Metallsalzen kontaktiert, so dass Metallionen von den Komplexbildnern komplexiert werden.

Gegenstand der vorliegenden Erfindung ist zudem die Verwendung der erfindungsgemäßen Konjugate zur Herstellung einer pharmazeutischen Zusammensetzung, wie insbesondere einer therapeutischen Zusammensetzung oder einer bildverstärkenden Zusammensetzung.

Gegenstand der vorliegenden Erfindung ist auch eine pharmazeutische Zusammensetzung, insbesondere eine therapeutische oder eine bildverstärkende Zusammensetzung, zumindest enthaltend ein erfindungsgemäßes Konjugat.

Gegenstand der vorliegenden Erfindung ist auch ein Kit zur Herstellung einer pharmazeutischen Zusammensetzung, insbesondere einer therapeutischen oder bildverstärkenden Zusammensetzung, zumindest enthaltend ein erfindungsgemäßes Konjugat. Dieses Konjugat kann dann je nach Anwendungszweck beispielsweise mit einem geeigneten Wirkstoff umgesetzt werden zur Herstellung einer therapeutischen Zusammensetzung oder falls ein Komplexbildner vorhanden ist, mit Metallionen, die bildverstärkende und/oder therapeutische Wirkung haben.

Gegenstand der vorliegenden Erfindung ist auch die Verwendung des erfindungsgemäßen Konjugats zur Herstellung von Makromolekül-Konjugaten, wobei zwei oder mehrere erfindungsgemäße Konjugate an ein Makromolekül gebunden werden.

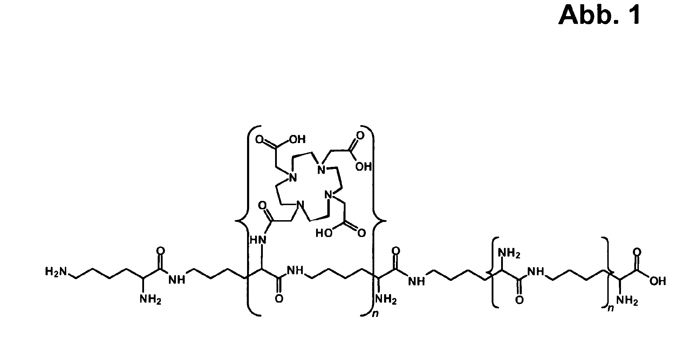

Abbildung 1 zeigt eine schematische Darstellung der in Synthese-Beispiel 1 erhaltenen erfindungsgemäßen Verbindung.

Abbildung 2 zeigt die Organverteilung von mit ¹¹¹In beladenen ε-Polylysin-DOTA. Nähere Angaben finden sich in Anwendungsbeispiel 1.

Abbildung 3 zeigt die chemische Struktur von MAG3, DMSA und DTPA.

Als bildverstärkende Zusammensetzung oder Kontrastmedium bzw. bildverstärkend wirken Stoffe, die in bestimmten diagnostischen Verfahren die Darstellung des Zielorgans verbessern, in der Regel indem der Kontrast zur Umgebung bzw. das Signal des Zielorgans im Verhältnis zur Umgebung erhöht wird.

Eine Carboxylgruppen tragende Verbindung gemäß der vorliegenden Erfindung ist eine chemische Verbindung, die zumindest eine Carboxylgruppe (-COOH) und zumindest eine Gruppe oder Funktionalität zur Anbindung an das Oligomer des erfindungsgemäßen Konjugats aufweist. Die Anbindung an das Oligomer kann auf jede bekannte Weise erfolgen, die zu einer kovalenten Verbindung von Oligomer und Carboxylgruppen tragender Verbindung führt. Beispiele für funktionelle Gruppen über die eine Anbindung erfolgen kann sind -NH₂, -SH, -OH, -Hal (z.B. -Cl, -Br, -I), -Alkin, -NCS, -NCO, -SO₂Cl, -Azid, -Carbonat, -Aldehyd, - Epoxid, -COOH, -COOR, wobei R in diesem Fall bevorzugt ein Halogen oder bevorzugt ein Aktivator, d.h. eine gute Abgangsgruppe ist, wie z.B. N-Hydroxysuccinimid, Pentafluorphenyl oder *para*-Nitrophenyl. Einen Überblick über mögliche kovalente Kopplungsarten findet man z.B. in "Bioconjugate Techniques", Greg T. Hermanson, Academic Press, 1996 auf den Seiten 137 bis 165.

Bevorzugt weist die Carboxylgruppen tragende Verbindung zwei oder mehr Carboxylgruppen auf. Beispiele für erfindungsgemäß geeignete Carboxylgruppen tragende Verbindungen sind: Citronensäure, Bernsteinsäure, Fumarsäure, Maleinsäure, Glutarsäure, Adipinsäure, Weinsäure, die folgenden Säuren habe ich unter dem Begriff Polycarbonsäuren gefunden, bitte prüfen, welche wahrscheinlich geeignet sind, Oxalsäure, Malonsäure, Bemsteinsäure, Glutarsäure, Adipinsäure, Korksäure, Azelainsäure, Sebacinsäure, die entsprechenden verzweigten Fettsäuren, Maleinsäure, Fumarsäure, Cyclohexandicarbonsäure und die entsprechenden Stellungsisomere und ähnliche aliphatische zweibasige Säuren; Tetrahydrophthalsäure, 5-Norbornen-2,3-dicarbonsäure und ähnliche alicyclische zweibasige Säuren; Tricarballylsäure, Aconitsäure, Trimesinsäure und ähnliche dreibasige Säuren; Adamantantetracarbonsäure, Butantetracarbonsäure, Cyclopentantetracarbonsäure, Tetrahydrofurantetracarbonsäure und ähnliche vierbasige Säuren; Zuckersäuren, insbesondere Aldarsäuren wie z. B. Glucarsäure, Galactarsäure; Äpfelsäure, Weinsäure, Citronensäure und ähnliche Hydroxyfettsäuren; Trimellitsäure, Pyromellitsäure,
Biphenyltetracarbonsäure, Benzophenontetracarbonsäure,
Diphenylsulfontetracarbonsäure und ähnliche aromatische Polycarbonsäuren.
Erfindungsgemäß gelten als Carboxylgruppen tragende Verbindung auch Komplexbildner, die mindestens eine Carboxylgruppe, bevorzugt zwei oder mehr Carboxylgruppen, aufweisen. Beispiele hierfür sind NOTA, TETA, EDTA oder bevorzugt DOTA oder DTPA. In diesem Fall erfüllen die Carboxylgruppen tragenden Verbindungen auch gleichzeitig die Funktion eines Komplexbildners, was insbesondere für diagnostische Anwendungen vorteilhaft ist.

Bevorzugte Carboxylgruppen tragende Verbindungen sind solche, die nach Konjugation an das Oligomer zwei oder mehr freie Carboxylgruppen aufweisen.

Ein Spacer, oft auch als Linker bezeichnet, bewirkt eine kovalente Bindung zwischen zwei Teilen eines Moleküls, im vorliegenden Fall beispielsweise zwischen dem erfindungsgemäßen Oligomer und einer Carboxylgruppen tragenden Verbindung oder einem Wirkstoff. Ein Spacer wird in der Regel dann eingeführt, wenn die Verbindung zwischen zwei Molekülteilen nicht nur über eine direkte chemische Bindung erfolgen soll, sondern ein gewisser Abstand zwischen zwei Molekülteilen erzeugt werden soll. Genauso kann ein Spacer die chemischen Funktionalitäten zur Verfügung stellen, die notwendig sind, um zwei Teile eines Moleküls, die ansonsten nicht miteinander reagieren würden, zu verbinden. Bevorzugt erfolgt die Konjugation eines Spacers an das Oligomer, die Carboxylgruppen tragende Verbindung oder einen Wirkstoff über eine Amid- oder eine Esterbindung. Spacer können beispielsweise aliphatische Kohlenwasserstoffe, Polyether (wie Polyethylenglycole), Oligopeptide oder ähnliche Elemente mit Ketten-Struktur sein. Der Spacer kann stabil sein, d.h. er ist unter physiologischen Bedingungen nicht oder nur geringfügig spaltbar, oder er kann instabil sein, d.h. er ist zumindest unter bestimmten physiologischen Bedingungen spaltbar.

Wirkstoffe, Peptide, Komplexbildner oder andere Funktionalitäten können direkt oder mittels eines Spacers an das Oligomer gebunden sein. Beispielsweise können an das erfindungsgemäße Konjugat Wirkstoffe über einen spaltbaren Linker gebunden werden. Dieser Linker wird dann unter bestimmten Bedingungen, z.B. durch enzymatische oder chemische Spaltung in vivo gespalten und setzt den Wirkstoff frei. Für diesen Zweck sind Linker, die Carbonsäureester- und Disulfidbindungen enthalten, geeignet, worin die ersteren Gruppen enzymatisch oder chemisch hydrolysiert werden und die letzteren durch Disulfidaustausch, z. B. in der Gegenwart von Glutathion, abgetrennt werden.

Ein Beispiel für einen spaltbaren Spacer ist auch ein Oligopeptid, welches gezielt mit Hilfe von speziellen, körpereigenen oder aber auch dem Körper zugesetzten Enzymen gespalten werden kann. So wird z.B. die Peptidsequenz DEVD (Asp-Glu-Val-Asp) nach Apoptoseinduktion durch Caspase-3 gespalten. Damit kann beispielsweise ein Wirkstoff oder eine Carboxylgruppen tragende Verbindung, die über einen derartigen Spacer gebunden ist, nach einer bestimmten Verweilzeit in der Niere aus selbiger entfernt werden, oder aber auch eine entsprechende Funktionalität (Vorhandensein oder Abwesenheit eines bestimmten Enzyms) der Niere überprüft werden. Weitere Beispiele sind die Peptidsequenzen CPEN↓FFWGGGG oder PENFF die durch die Matrix-Metalloprotease-13 gespalten werden kann. Eine einfache Ausführungsform eines spaltbaren Spacers ist die Bildung eines Carbonsäureesters, der durch Esterasen leicht gespalten werden kann.

Alternativ kann der Spacer eine säurelabile Struktur, z.B. ein Hydrazon, ein Imin, ein Carboxylhydrazon, ein Acetal oder Ketal enthalten (siehe beispielsweise Haag-R, Kratz-F, Angewandte Chemie Seite 1218 (2006)).

Erfindungsgemäß sind Aminosäuren Verbindungen, die mindestens eine Aminogruppe und mindestens eine Carboxylgruppe tragen. Beispiele sind natürliche, proteinogene Aminosäuren oder nicht-proteinogene in Organismen vorkommende oder synthetisch hergestellte Aminosäuren.

Ein Peptid ist eine Verbindung, die aus einer Verknüpfung zweier oder mehrerer Aminosäuren entstanden ist. Dabei sind die einzelnen Aminosäuren in einer definierten Reihenfolge (Sequenz) zu einer, meist unverzweigten, Kette verbunden. Die Aminosäuren in Peptiden und in den größeren Proteinen sind über Amidbindungen miteinander verbunden.

Eine Festphase ist erfindungsgemäß ein organisches, anorganisches oder organisch/anorganisches Verbundmaterial, das als Harz oder Träger in der Festphasensynthese eingesetzt werden kann. Des Weiteren gelten erfindungsgemäß als Festphase auch Oberflächen von Formkörpern wie z.B. Mikrotiterplatten oder partikuläre Materialien, wie z.B. organische oder anorganische Nanopartikel, Metallpartikel oder ähnliches.

Erfindungsgemäß sind Wirkstoffe oder Wirkstoffmoleküle entsprechend dem deutschen Arzneimittelgesetz Stoffe, die dazu bestimmt sind, bei der Herstellung von Arzneimitteln als arzneilich wirksame Bestandteile verwendet zu werden oder bei ihrer Verwendung in der Arzneimittelherstellung zu arzneilich wirksamen Bestandteilen zu werden (AMG § 4 (19)). Wirkstoffe rufen in der Regel in einem Organismus eine spezifische Wirkung hervor. Ein erfindungsgemäßer Wirkstoff ist typischerweise ein pharmazeutisch wirksames Molekül oder Arzneimittel, wie etwa **Immunsuppressiva** z. B. Azathioprin, Mycophenolat-Mofetil, Ciclosporin, Tacrolimus, Sirolimus, Fingolimod oder Triptolid, **Zytostatika** z.B. Bleomycin, Dactinomycin, Mitomycin, Daunorubicin, Doxorubicin, Epirubicin, Idarubicin, Mitoxantron, Amascrin, Doxofluridin, Cisplatin, Carboplatin, Oxaliplatin, Satraplatin, Camptothecin, Toptecan, Irinotecan, Amsacrin, Etoposid, Teniposid, Cyclophosphamid, Trofosfamid, Melphalan, Chlorambucil, Estramustin, Busulfan, Chlorambucil, Chlormethin, Treosulfan, Carmustin, Lomustin, Nimustin, Procarbazin, Streptozocin, Dacarbazin, Ifosfamid, Temozolomid, Thiotepa, Vinorelbin, Vincristin, Vinblastin, Vindesin, Paclitaxel, Docetaxel, Methotrexat, Pemetrexed, Raltitrexed, Fluorouracil, Capecitabin, Cytosinarabinosid, Gemcitabin, Tioguanin, Pentostatin, Mercaptopurin, Fludarabin, Caldribin, Hydroxycarbamid, Mitotan, Azacitidin, Cytarabin, Nelarabin, Bortezomib, Anagrelid insbesondere der Protein-Kinase-Inhibitoren wie z. B. Imatinib, Erlotinib, Sunitinib, Sorafenib, Dasatinib, Lapatinib oder Nilotinib, **Immuntherapeutika** z. B. Cetuximab, Alemtuzumab und Bevacizumab, **Antiphlogistika** z. B. Naproxen, Ibuprofen, Indometacin, Prednisolon, Prednison, Hydrocortison oder Budesonid, **Antibiotika** insbesondere der Penicilline wie z. B. Benzylpenicillin, Methicillin oder Amoxicillin, der Cephalosporine wie z. B. Cefuroxim, Cefotaxim, Cefadroxil oder Cefixim, der β-Lactamase-Inhibitoren wie z. B. Clavulansäure, Sulbactam oder Tazobactam, der Carbapeneme wie z. B. Imipenem oder Meropenem, der Monobactame wie z. B. Aztreonam, der Tetracycline wie z. B. Tetracyclin, Chlortetracyclin, Oxytetracyclin, Doxycyclin, Minocyclin oder Tigecyclin, der Makrolid-Antibiotika wie z. B. Erythromycin A, der Glykopeptid-Antibiotika wie z. B. Vancomycin, der Endiine wie z.B. Calicheamicin, **Virostatika** z. B. Aciclovir, Valaciclovir, Ganciclovir, Valganciclovir, Penciclovir, Famciclovir, Brivudin, Cidofovir, Foscarnet, Idoxuridin oder Tromantadin, **Antihypertensiva** insbesondere der **ACE-Hemmer** wie z. B. Benazepril, Captopril, Cilazapril, Enalapril, Fosinopril, Lisinopril, Perindopril, Quinapril, Ramipril, Trandolapril oder Zofenopril, der **Sartane** wie z. B. Losartan, Balsartan, Irbesartan, Candesartan, Eprosartan, Olmesartan oder Telmisartan, der **Renin-Inhibitoren** wie z. B. Aliskiren und der **Betablocker** wie z. B. Proproanolol, Pindolol, Sotalol, Bopindolol, Atenolol, Bisorpolol, Celiprolol, Esmolol, Metoprolol, Nebivolol, Oxprenolol, Carvedilol oder Labetalol, **Urikosurika** z. B. Probenecid oder Benzbromaron oder **Diuretika** z. B. Acetazolamid, Furosemid, Torasemid, Bumetanid, Piretanid, Azosemid, Etacrynsäure, Etozolin, Hydrochlorothiazid, Benzthiazid, Chlorothiazid, Chlorthalidon, Indapamid, Mefrusid, Metolazon, Clopamid, Xipamid, Hydroflumethiazid, Methyclothiazid, Polythiazid, Amilorid, Triameteren, Spironolacton, Canrenon, Eplerenon oder Spironolacton.

Weitere Antitumormittel, z. B. Mittel, die gegen proliferierende Zellen wirksam sind, sind erfindungsgemäß ebenfalls Wirkstoffe. Beispielhafte Antitumormittel schließen Cytokine wie etwa Interleukin-2 (IL-2), Tumornekrosefaktor oder dergleichen, Lectin-Entzündungsreaktionspromotoren (Selectine) wie etwa L-Selectin, E-Selectin, P-Selectin oder dergleichen, und ähnliche Moleküle ein.

Erfindungsgemäß können pro erfindungsgemäßem Konjugat ein oder mehrere gleiche oder unterschiedliche Wirkstoffmoleküle gebunden sein.

Bei Makromolekülen, d.h. z.B. bei größeren Wirkstoffmolekülen, insbesondere bei Proteinen, können umkehrt zwei oder mehrere, bevorzugt 2, 3, 4, 5, 6, 7, 8, 9 oder 10, erfindungsgemäße Konjugate an ein Wirkstoffmolekül gebunden werden, um eine nierenspezifische Anreicherung des Wirkstoffs zu ermöglichen. Typischerweise erfolgt auch hier eine kovalente Anbindung der erfindungsgemäßen Konjugate an das Makromolekül. Als Makromoleküle gelten erfindungsgemäß nicht nur große Moleküle wie Proteine sondern auch jede Form von Partikeln (z.B. Nanopartikel), Liposomen oder ähnliches.

Zusätzlich zu den Wirkstoffmolekülen oder statt der Wirkstoffmoleküle können an das erfindungsgemäße Konjugat auch andere Funktionalitäten, wie beispielsweise Funktionalitäten für diagnostische oder bildgebende Verfahren, gebunden sein.

Genauso können als Funktionalität über optionale Spacer fluor-haltige Seitenketten eingebaut werden. Mit Hilfe der ¹⁹F-Kernresonanztomographie kann so die Anreicherung der entsprechenden Moleküle in den Nieren dargestellt werden. Besonders vorteilhaft sind dabei hochsymmetrisch angeordnete Fluoratome, die eine einheitliche Resonanzfrequenz aufweisen. Zur Verbesserung des ¹⁹F-Signals kann ein in der Kernspintomographie übliches Kontrastmittel wie beispielsweise Gadobutrol (Magnevist^{®}), verwendet werden.

Falls das erfindungsgemäße Konjugat Komplexbildner enthält, ist es besonders vorteilhaft Gadolinium oder Mangan oder ein anderes dem Fachmann bekanntes stark paramagnetisches Metallion mit Hilfe eines am erfindungsgemäßen Konjugat befindlichen Komplexbildners zu integrieren. Geeignete Komplexbildner sind dabei beispielsweise DOTA und DTPA.

Weiterhin können - auch optional über Spacer - Komplexbildner konjugiert werden, die nicht in die Gruppe der Carboxylgruppen tragenden Verbindungen gehören. Beispiele sind Hydroxychinolin-, Thioharnstoff-, Guanidin-, Dithiocarbamat-, Hydroxamsäure-, Amidoxim-, Aminophosphorsäure-, (cyclische) Polyamino-, Mercapto-, 1,3-Dicarbonyl-und Kronenether-Reste mit zum Teil sehr spezifischen Aktivitäten gegenüber Ionen unterschiedlicher Metalle.

Auch können Funktionalitäten für zellspezifisches Targeting wie z. B. Antikörper, Antikörperfragmente oder Aptamere an das erfindungsgemäße Konjugat gebunden sein. Auch Fluoreszenzfarbstofffe oder Interleukine wie IL-2 können gebunden sein.

"Peptidisch verknüpft" bedeutet erfindungsgemäß, dass eine -NH-CO-Bindung zwischen zwei Monomereinheiten vorliegt, wie sie auch in Peptiden oder Proteinen zwischen zwei Aminosäuren als Monomereinheiten vorliegt. Das bedeutet, zwei Monomereinheiten sind derart verknüpft, dass eine -NH-Gruppe des einen Monomeren mit einer - C=O-Gruppe des anderen Monomeren verknüpft ist. Es entsteht demnach folgende Bindungsstruktur:

M-NH-CO-M-NH-CO-M-NH-CO-M, wobei M der Teil des Monomers ist, der nicht an der Bindung beteiligt ist.

Das Konjugat der vorliegenden Erfindung besteht zumindest aus zwei kovalent miteinander verbundenen Teilen - einer Carboxylgruppen tragenden Verbindung und einem Oligomer. In einer bevorzugten Ausführungsform enthält das Konjugat ein Oligomer, ein oder mehrere Carboxylgruppen tragende Verbindungen und ein oder mehrere Wirkstoffmoleküle. In einer besonders bevorzugten Ausführungsform enthält das erfindungsgemäße Konjugat mehrere, d.h. beispielsweise 2, 3, 4, 5, 6, 7, 8, 9 oder 10 gleiche oder unterschiedliche Carboxylgruppen tragende Verbindungen und mehrere, d.h. beispielsweise 2, 3, 4, 5, 6, 7, 8, 9, oder 10 gleiche oder unterschiedliche Wirkstoffmoleküle.
Ein Komplexbildner ist erfindungsgemäß jede Molekülstruktur, die in der Lage ist, Metallionen zu komplexieren, d.h. mit den Metallionen einen Metall-Chelat-Komplex zu bilden. Komplexbildner werden oft auch als Chelatoren bezeichnet. Beispiele für erfindungsgemäß geeignete Komplexbildner sind EDTA, NOTA, TETA, Iminodiessigsäure, DOTA oder DTPA. Besonders bevorzugt sind erfindungsgemäß Komplexbildner, die Metallionen binden, die in SPECT, PET, CT oder MRT-Messungen detektiert werden können. Bevorzugte Komplexbildner sind DOTA oder DTPA oder deren Derivate. Komplexbildner sind erfindungsgemäß sowohl Moleküle, an die die Metallionen bereits gebunden sind, wie auch Moleküle, an die Metallionen binden können, aber im vorliegenden Stadium nicht gebunden sind.

Erfindungsgemäß geeignete Metallionen zur Bindung an Komplexbildner sind z.B. Fe²⁺, Fe³⁺, Cu²⁺, Cr³⁺, Gd³⁺, Eu³⁺, Dy³⁺, La³⁺, Yb³⁺ und/oder Mn²⁺ oder auch die Ionen von Radionukliden, wie Gamma-Emitter, Positronen-Emitter, Auger-Elektronen-Emitter, Alpha-Strahler und Fluoreszenz-Emitter, z.B. ⁵¹ Cr, ⁶⁷ Ga, ⁶⁸ Ga, ¹¹¹ In, ^{99m} Tc, ¹⁴⁰ La, ¹⁷⁵ Yb, ¹⁵³ Sm, ¹⁶⁶ Ho, ⁸⁸Y, ⁹⁰Y, ¹⁴⁹ Pm, ¹⁷⁷ Lu, ⁴⁷ Sc, ¹⁴² Pr, ¹⁵⁹ Gd, ²¹² Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹⁹⁷Hg, ²¹¹At, ¹⁶⁹Eu, ²⁰³Pb, ²¹²Pb, ⁶⁴Cu, ⁶⁷Cu, ¹⁸⁸Re, ¹⁸⁶Re, ¹⁹⁸Au und/oder ¹⁹⁹Ag_{.}

Beispiele für geeignete Metallionen und ihre jeweilige Anwendung sind:
- ¹¹¹In für SPECT
- ⁶⁸Ga für PET
- ⁹⁰Y zur Therapie
- Gd, Eu, Mn für MRT
- Gadolinium, Wolfram oder andere Elemente mit hoher Ordnungszahl für die Computertomographie

Als Oligomer wird erfindungsgemäß der Teil des Konjugats bezeichnet, der aus einem Oligomer besteht, das aus peptidisch verknüpften Monomereinheiten besteht. Das Oligomer besteht typischerweise aus 5 bis 1000, bevorzugt aus 8 bis 100, besonders bevorzugt aus 10 bis 50 Monomereinheiten. In einer besonders bevorzugten Ausführungsform besteht das Oligomer aus ε-Polylysin, das zwischen 8 und 100 Monomereinheiten, besonders bevorzugt zwischen 10 und 50 Monomereinheiten aufweist.

In anderen Ausführungsformen können jedoch bis zu 50% der ε-Lysin-Monomereinheiten durch andere Monomereinheiten ersetzt sein und/oder bis zu 50% der ε-Lysin-Monomereinheiten durch Einführung weiterer Funktionalitäten derivatisiert bzw. modifiziert sein. Genauso kann das Oligomer, das aus peptidisch verknüpften Monomereinheiten besteht, mehrere aufeinanderfolgende Monomereinheiten enthalten, die nicht ε-Lysin-Monomereinheiten sind, wenn es mindestens 10 aufeinander folgende Monomereinheiten enthält, die zu mindestens zu 70% (bezogen auf die Anzahl der Monomereinheiten) bevorzugt zu mindestens 80% aus ε-Lysin-Einheiten bestehen. Dies ist beispielsweise der Fall, wenn sich an einem Ende des Oligomers eine Kette von 10 bis 20 Monomereinheiten (z.B. aus Aminosäuren) befindet, in der keine ε-Lysin-Monomereinheit enthalten ist und daran anschließend beispielsweise zehn Monomereinheiten, von denen acht ε-Lysin-Monomereinheiten sind und zwei aus anderen Aminosäuren bestehen.

Als Monomereinheit gilt erfindungsgemäß jeder Teil des Oligomers, der mit mindestens einem weiteren Teil des Oligomers peptidisch verknüpft ist. Endständige Monomereinheiten sind dabei in der Regel nur mit einer weiteren Monomereinheit peptidisch verknüpft. Monomereinheiten in der Mitte des Oligomers sind mit zwei weiteren Monomereinheiten peptidisch verknüpft. An Verzweigungsstellen befinden sich Monomereinheiten, die mit drei weiteren Monomeren peptidisch verknüpft sind. Bei Monomereinheiten in der Mitte des Oligomers stellt die Monomereinheit typischerweise einmal den -NH-Teil der peptidischen Bindung und einmal den -CO-Teil zur Verfügung.

Eine ε-Lysin-Monomereinheit ist erfindungsgemäß eine ε-Lysin-Einheit, eine Ornithin-Einheit, eine 2,3-Diaminopropionsäure-Einheit oder eine 2,4-Diaminobuttersäure-Einheit. Bevorzugt besteht die ε-Lysin-Monomereinheit aus einer ε-Lysin-Einheit. Der Begriff Einheit soll dabei jeweils zeigen, dass es sich um eine Einheit bzw. ein Monomer in einem peptidisch verknüpften Oligomer handelt und nicht um die freie Aminosäure.

Eine ε-Lysin-Einheit hat folgende chemische Struktur:

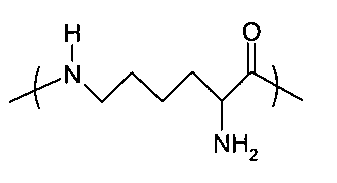

Die ε-Lysin-Monomereinheiten können in dem erfindungsgemäßen Oligomer in der D oder L-Form vorliegen.

Typische andere Monomereinheiten, die das erfindungsgemäße Oligomer neben den ε-Lysin-Monomereinheiten enthalten kann, sind natürliche oder synthetische Aminosäuren, wie insbesondere Alanin, β-Alanin, Glycin, Glutaminsäure, Asparaginsäure oder Arginin.
Weitere typische Monomereinheiten sind Monomereinheiten mit Spacerfunktion der Formel

-NH-SP-CO- I

wobei SP eine C1 bis C20 Alkylen-, Alkenylen- oder Alkinylen-Gruppe sein kann, bei der ein oder mehrfach nicht benachbarte Methylengruppen ersetzt sein können durch -O-, -S-, -S(O)-, -SO₂-, -SO₂O-, -C(O)-, -C(O)O-, -CH₂-, -CHR'-, -CR'₂-, -CR'=CH-, -CH-CR'-, -CH=CH-, -CR'=CR'-, -C≡C-, - N⁺R'₂-, -P(O)R'O-, -C(O)NR'-, -SO₂NR'-, -OP(O)R'O-, -P(O)(NR'₂)NR'-, - PR'₂=N- oder -P(O)R'-
mit R' = C₁- bis C₆-Alkyl, C₃- bis C₇-Cycloalkyl, unsubstituiertes oder substituiertes Phenyl.
SP steht bevorzugt für lineare C3 bis C10-Alkyl-Ketten, lineare C3-C10-Ketten mit ein oder mehreren Alkylengruppen, für Ethylenglycol-Ketten mit zwei bis zehn Ethylenglycoleinheiten oder für Oligopeptidketten.

Weitere typische Monomereinheiten sind solche, die Funktionalitäten zur Anknüpfung von Spacern, Wirkstoffen, Komplexbildnern, Peptiden, Farbstoffen, Lösungsvermittlern, Schutzgruppen, einer Festphase oder ähnlichen Komponenten enthalten oder an die bereits Komponenten, wie Wirkstoffe, Komplexbildner, Peptide, Lösungsvermittler, Schutzgruppen, eine Festphase oder Farbstoffe direkt oder über einen Spacer angebunden sind. Derartige Monomereinheiten haben bevorzugt zumindest eine der folgenden funktionellen Gruppen -NH₂, -SH, -OH, -Hal (z.B. -Cl, -Br, -I), - Alkin, -NCS, -NCO, -SO₂Cl, -Azid, -Carbonat, -Aldehyd, -Epoxid, -COOH, - COOR, wobei R in diesem Fall bevorzugt ein Halogen oder bevorzugt ein Aktivator, d.h. eine gute Abgangsgruppe ist, wie z.B. N-Hydroxysuccinimid, Pentafluorphenyl oder *para*-Nitrophenyl, oder sind über eine solche funktionelle Gruppe mit Wirkstoffen, Komplexbildnern, Peptiden, Farbstoffen oder ähnlichen Komponenten verknüpft.

Weiterhin kann das erfindungsgemäße Oligomer ε-Lysin-Monomereinheiten enthalten, die derivatisiert sind. Dies sind Monomereinheiten, in denen entsprechend an der NH-Gruppe und/oder der Aminogruppe weitere Funktionalitäten (F1/F2) gebunden sind. Dabei kann es sich bei F1 und F2 unabhängig voneinander um eine Acetylgruppe handeln oder auch um direkt oder über einen Spacer gebundene Wirkstoffe, Komplexbildner, Peptide, Farbstoffe, Lösungsvermittler, Schutzgruppen, eine Festphase oder ähnlichen Komponenten. Eine entsprechende ε-Lysin-Einheit, in der mit den Gruppen F1 und/oder F2 eine Derivatisierung eingeführt ist, ist in Formel II dargestellt.

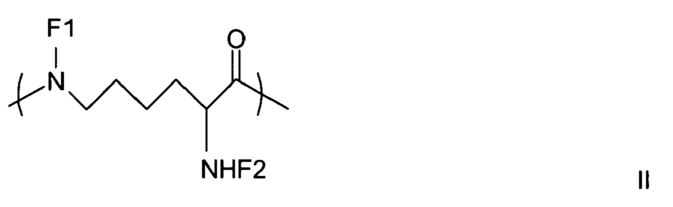

Für den Fachmann ist es offensichtlich, dass die oben dargestellten Formeln Monomereinheiten in der Mitte der Oligomerkette darstellen und dass endständige Monomereinheiten in Abhängigkeit davon, ob sie sich am C- oder N-terminalen Ende befinden jeweils statt -CO- eine COOH oder COOR-Gruppe tragen bzw. statt -NH- bzw. -NF1- eine NH₂, NF1 H, NF1 R, NHR oder NR₂-Gruppe tragen, wobei R typischerweise H, lineares oder verzweigtes C1-C6 Alkyl, eine Spacerfunktion zur Anbindung von Wirkstoffen, Komplexbildnern, Peptiden, Farbstoffen, Lösungsvermittlern, Schutzgruppen, einer Festphase oder ähnlichen Komponenten oder ein direkt oder über einen Spacer gebundener Wirkstoff, Komplexbildner, Peptid, Farbstoff, Lösungsvermittler, Schutzgruppe, eine Festphase oder ähnliche Komponente ist.

Dementsprechend sind ε-Polylysin-Derivate erfindungsgemäße Oligomere, die nicht vollständig aus ε-Lysin-Einheiten aufgebaut sind, sondern in denen anderen ε-Lysin-Monomereinheiten wie z.B. Ornithin-Einheiten vorkommen und/oder in denen entsprechend der erfindungsgemäßen Vorgaben ein Teil der Monomereinheiten beispielsweise aus anderen Aminosäuren als ε-Lysin, Ornithin, 2,3-Diaminopropionsäure oder 2,4-Diaminobuttersäure aufgebaut ist und/oder aus Verbindungen der Formel I.

Die vorliegende Erfindung basiert auf dem überraschenden Effekt, dass Konjugate von ε-Polylysin und ε-Polylysin-Derivaten mit Carboxylgruppen tragenden Verbindungen z.B. nach Injektion in die Blutbahn oder nach subkutaner Injektion nahezu ausschließlich in der Niere angereichert werden. Dementsprechend sind die erfindungsgemäßen Konjugate für den Einsatz in therapeutischen Verfahren zur Behandlung der Niere, in bildgebenden Verfahren zur Darstellung der Niere sowie zum Nieren-Targeting geeignet.

Bevorzugt werden die erfindungsgemäßen Verbindungen ausgehend von ε-Polylysin hergestellt, indem die entsprechenden Carboxylgruppen tragenden Verbindungen und bevorzugt ein oder mehrere Wirkstoffmoleküle oder weitere Funktionalitäten optional nach entsprechender Aktivierung konjugiert werden.

ε-Polylysin ist ein Homopolymer der Aminosäure L-Lysin. ε-Polylysin wird von dem Bakterium *Streptomyces albulus* in einem aeroben Prozess produziert. Dieses natürlich hergestellte ε-Polylysin enthält ungefähr 30 L-Lysin-Einheiten. ε-Polylysin ist in Japan als antimikrobielles Konservierungsmittel für Lebensmittel zugelassen. ε-Polylysin wird im Gegensatz zu α-Polylysin von Proteasen nicht enzymatisch abgebaut. Erfindungsgemäß kann jede Art von ε-Polylysin eingesetzt werden, d.h. z.B. natürlich hergestelltes, gentechnisch hergestelltes oder synthetisch hergestelltes ε-Polylysin. Die chemische Synthese von ε-Polylysin erfolgt entsprechend der herkömmlichen Peptid-Synthese mit entsprechend geschütztem L-Lysin, so dass die Peptid-Bindung über die ε-Aminogruppe der Seitenkette erfolgt.

Zur Herstellung der erfindungsgemäßen Verbindungen kann ε-Polylysin einer bestimmten Kettenlänge eingesetzt werden (z.B. synthetisch hergestelltes ε-Polylysin oder aufgereinigtes natürliches ε-Polylysin) oder auch eine Mischung von ε-Polylysin verschiedener Kettenlänge, wie es z.B. natürlich aus *Streptomyces albulus* erhalten wird. Erfindungsgemäß fällt daher sowohl ε-Polylysin einer bestimmten Kettenlänge als auch Mischungen von ε-Polylysin verschiedener Kettenlänge oder Mischungen enthaltend verschiedene ε-Polylysin-Derivate unter den Begriff ε-Polylysin und ε-Polylysin-Derivate.

Es wurde weiterhin gefunden, dass nicht nur Konjugate des ε-Polylysin eine hervorragende Anreicherung in der Niere zeigen, sondern auch Konjugate aus ε-Polylysin-Derivaten.

Typische erfindungsgemäß geeignete Konjugate lassen sich auch durch Formel III darstellen

A-(Lys)ₙ-E III

worin
n eine Zahl zwischen 5 und 1000 ist,
A
- eine oder mehrere geladene oder ungeladene direkt oder über einen Spacer oder eine dendritische Funktionalität gebundene Endgruppen, z.B. Wasserstoff, -CN, -OR', -NR'₂, -P(O)R'₂, -P(O)(OR')₂, -P(O)(NR'₂)₂, - C(O)R', -C(O)OR', -C(O)OH, -C(O)NR'₂, -SO₂NR'₂, -C(O)Hal, SO₂OH, - SO₂Hal, -NO₂, -Hal oder
- ein oder mehrere direkt oder über einen Spacer oder eine dendritische Funktionalität gebundene Gruppen, wie z.B. Carboxylgruppen tragende Verbindungen, Wirkstoffmoleküle, Komplexbildner, Farbstoffe, eine oder mehrere gleiche oder unterschiedliche Aminosäuren, Peptide, Proteine, Lösungsvermittler, Schutzgruppen oder eine Festphase
E
- ein oder mehrere geladene oder ungeladene direkt oder über einen Spacer oder eine dendritische Funktionalität gebundene Endgruppen, z.B. Wasserstoff, -CN, -OR', -NH₂, NHR', -NR'₂, -P(O)R'₂, -P(O)(OR')₂, - P(O)(NR'₂)₂, -C(O)R', -C(O)OR', -C(O)OH, -C(O)NR'₂, -SO₂NR'₂, -C(O)Hal, SO₂OH, -SO₂Hal, -NO₂, -Hal oder
- ein oder mehrere direkt oder über einen Spacer oder eine dendritische Funktionalität gebundene Gruppen, wie z.B. Carboxylgruppen tragende Verbindungen, Wirkstoffmoleküle, Komplexbildner, Farbstoffe, eine oder mehrere gleiche oder unterschiedliche Aminosäuren, Peptide, Proteine, Lösungsvermittler, Schutzgruppen oder eine Festphase,
Lys unabhängig voneinander
- eine ε-Lysin-Monomereinheit entsprechend der bereits gegebenen Definition
- eine Monomereinheit entsprechend Formel I oder II
- eine Gruppierung entsprechend Formel IV

   (NH-M-CO) IV

   wobei M unabhängig voneinander
   -, -O-, -S-, -S(O)-, -SO₂-, -SO₂O-, -C(O)-, -C(O)O-, -CH₂-, -CHR'-, -CR'₂-, - CR'=CH-, -CH-CR'-, -CH=CH-, -CR'=CR'-, -C≡C-, -N⁺R'₂-, -P(O)R'O-, - C(O)NR'-, -SO₂NR'-, -OP(O)R'O-, -P(O)(NR'₂)NR'-, -PR'₂=N- oder -P(O)R'-bedeuten kann oder
- geradkettiges oder verzweigtes Alkyl mit 1 bis 20 C-Atomen,
- geradkettiges oder verzweigtes Alkenyl mit 2-20 C-Atomen und einer oder mehreren Doppelbindungen,
- geradkettiges oder verzweigtes Alkinyl mit 2-20 C-Atomen und einer oder mehreren Dreifachbindungen,
- gesättigtes, teilweise oder vollständig ungesättigtes Cycloalkyl mit 3-7 C-Atomen, das mit Alkylgruppen mit 1-6 C-Atomen substituiert sein kann, wobei ein oder mehrere nicht benachbarte Methylengruppen ersetzt sein können durch -O- oder -S- und benachbarte Methylengruppen ersetzt sein können durch Alkenyl- oder Alkinylgruppen,
wobei ein oder mehrere Methylengruppen unabhängig voneinander ersetzt sein können durch -O- , -S-, -S(O)-, -SO₂-, -SO₂O-, -C(O)-, -C(O)O-, -CH₂-, -CHR'-, -CR'₂-, -CR'=CH-, -CH-CR'-, -CH=CH-, -CR'=CR'-, -C≡C-, -N⁺R'₂-, -P(O)R'O-, -C(O)NR'-, -SO₂NR'-, -OP(O)R'O-, -P(O)(NR'₂)NR'-, -PR'₂=N-oder -P(O)R'-
und ein oder mehrere in M enthaltenen Methylengruppen unabhängig voneinander ein oder zweifach mit R" substituiert sein können, wobei
R' lineares oder verzweigtes C₁- bis C₈-Alkyl, C₃- bis C₇-Cycloalkyl, unsubstituiertes oder substituiertes Phenyl und
R" lineares oder verzweigtes C₁- bis C₈-Alkyl, C₃- bis C₇-Cycloalkyl, unsubstituiertes oder substituiertes Phenyl
oder -CN, -OR', -NH₂, NHR', -NR'₂, -P(O)R'₂, -P(O)(OR')₂, -P(O)(NR'₂)₂, - C(O)R', -C(O)OR', -C(O)OH, -C(O)NR'₂, -SO₂NR'₂, -C(O)Hal, SO₂OH, - SO₂Hal, -NO₂, -Hal ist und
Hal = -F, -Cl, -Br oder -I,
wobei an alle zur Konjugation geeigneten funktionellen Gruppen (z.B. NH, NH₂, COOH, OH, -SH, -Hal (z.B. -Cl, -Br, -I), -Alkin, -Azid, -Aldehyd) der Monomereinheiten Lys unabhängig voneinander Carboxylgruppen tragende Verbindungen, Wirkstoffe, Komplexbildner, Peptide, Lösungsvermittler, Schutzgruppen, eine Festphase, Farbstoffe oder ähnliche Komponenten direkt oder über einen Spacer angebunden sein können,
mit der Maßgabe, dass die erfindungsgemäße Verbindung mindestens eine Carboxylgruppen tragende Verbindung aufweist und entweder mehr als 50% der Monomereinheiten (bezogen auf die Anzahl der Monomereinheiten) ε-Lysin-Monomereinheiten sind oder mindestens 10 aufeinander folgende Monomereinheiten mindestens zu 70% (bezogen auf die Anzahl der Monomereinheiten) ε-Lysin-Monomereinheiten sind.

In einer bevorzugten Ausführungsform der erfindungsgemäßen Verbindung ist A
-C(O)OR', -C(O)OH, -C(O)NR'₂ oder -C(O)Hal oder
ein oder mehrere direkt oder über einen Spacer oder eine dendritische Funktionalität gebundene Komponenten, wie Carboxylgruppen tragende Verbindungen, Wirkstoffe, Komplexbildner, Peptide, Lösungsvermittler, Schutzgruppen, eine Festphase oder Farbstoffe.

Besonders bevorzugt ist A - OH, - OCH₃, - OCH₂CH₃ oder ein oder mehrere direkt oder über einen Spacer oder eine dendritische Funktionalität gebundene Wirkstoffmoleküle.

In einer bevorzugten Ausführungsform ist E
-H, -CH₃, -CH₂CH₃ oder
oder
ein oder mehrere direkt oder über einen Spacer oder eine dendritische Funktionalität gebundene Komponenten, wie Carboxylgruppen tragende Verbindungen, Wirkstoffe, Komplexbildner, Peptide, Lösungsvermittler, Schutzgruppen, eine Festphase oder Farbstoffe.

Besonders bevorzugt ist E
ein oder mehrere direkt oder über einen Spacer oder eine dendritische Funktionalität gebundene Wirkstoffmoleküle.

Wenn die erfindungsgemäße Verbindung ein oder mehrere Gruppen Lys enthält, die eine NH₂- oder COOH-Gruppe aufweisen, so können darüber weitere Monomereinheiten über eine peptidische Bindung angebunden werden und es entstehen ein- oder mehrfach verzweigte Verbindungen. Bevorzugt sind die erfindungsgemäßen Verbindungen unverzweigt.

n ist bevorzugt eine Zahl zwischen 8 und 100, besonders bevorzugt zwischen 10 und 50.

Bevorzugt enthalten die erfindungsgemäßen Konjugate nicht nur eine sondern mehrere Carboxylgruppen tragende Verbindungen. Diese können direkt oder über einen Spacer an das Carboxy- und/oder Amino-terminale Ende des Oligomers gebunden sein und/oder an zur Konjugation geeignete funktionelle Gruppe der Monomereinheiten (z.B. NH, -NH₂, -COOH, -OH, - SH, -Hal (z.B. -Cl, -Br, -I), -Alkin, -Azid, -Aldehyd).

In einer bevorzugten Ausführungsform erfolgt die Anbindung der Carboxylgruppen tragenden Verbindungen über Aminogruppen der Monomereinheiten, z.B. die freie Aminogruppe des ε-Lysin.

Bevorzugt sollte das erfindungsgemäße Konjugat mindestens eine Carboxylgruppen tragende Verbindung pro 10 Monomereinheiten aufweisen, besonders bevorzugt zwischen 3 und 6 Carboxylgruppen tragende Verbindungen pro 10 Monomereinheiten. Genauso ist jedoch auch möglich, dass an mehr als 9 von 10 Monomereinheiten oder an alle Monomereinheiten eine Carboxylgruppen tragende Verbindung angebunden ist. Die optimale Anzahl der Carboxylgruppen tragenden Verbindungen pro 10 Monomereinheiten hängt von der Art der Carboxylgruppen tragenden Verbindung ab sowie der Art der Monomereinheiten. Die oben angegebene bevorzugte Anzahl von Carboxylgruppen tragenden Verbindungen pro Monomereinheit, gelten insbesondere für Oligomere, die vollständig aus ε-Lysin-Monomereinheiten aufgebaut sind. Die Verteilung der Carboxylgruppen tragenden Verbindungen in dem erfindungsgemäßen Konjugat kann dabei willkürlich sein, so dass z.B. die ersten Monomereinheiten -NH₂ aufweisen, dann eine Monomereinheit mit Carboxylgruppen tragender Verbindung folgt, dann einmal eine mit -NH₂, dann zwei mal eine Monomereinheit, die eine Carboxylgruppen tragende Verbindung trägt, dann wieder zweimal eine mit -NH₂, usw.

Genauso kann die Verteilung der Komplexbildner in der erfindungsgemäßen Verbindung auch geordnet sein, so dass z.B. jede zweite Monomereinheit eine daran gebundene Carboxylgruppen tragende Verbindung aufweist. Die Ordnung kann auch so erfolgen, dass an einem Ende des erfindungsgemäßen Konjugats alle Monomereinheiten eine konjugierte Carboxylgruppen tragende Verbindung aufweisen, während der Rest der Monomere freie NH₂ -Funktionen aufweist.

Bevorzugt liegen die Carboxylgruppen tragenden Verbindungen in den erfindungsgemäßen Konjugaten willkürlich verteilt vor.

Die erfindungsgemäßen Konjugate können insbesondere auf verschiedene, dem Fachmann im Bereich der Peptid-Synthese bekannten Verfahren hergestellt werden.

### 1. Synthese ausgehend von ε-Polylysin

Dabei wird typischerweise synthetisches oder natürliches ε-Polylysin einheitlicher oder unterschiedlicher Kettenlänge in Lösung mit den entsprechenden Carboxylgruppen tragenden Verbindungen zur Reaktion gebracht. Dazu können beispielsweise zunächst die Carboxylgruppen tragende Verbindungen aktiviert werden. Dies kann z.B. durch Aktivierung einer oder mehrerer ihrer Carboxylgruppen erfolgen, indem sie zum Aktivester oder Säurechlorid umgesetzt werden. Anschließend erfolgt die Umsetzung mit ε-Polylysin, wobei die Konjugation bevorzugt an die freien Aminogruppen erfolgt. Alternativ können beispielsweise ein oder mehrere Carboxylgruppen der Carboxylgruppen tragenden Verbindung mit einem Kopplungsreagenz wie Dicyclohexylcarbodiimid oder HATU aktiviert und mit dem ε-Polylysin umgesetzt werden, wobei die Konjugation bevorzugt an die freien Aminogruppen erfolgt. Reaktionsbedingungen für derartige Umsetzungen sind dem Fachmann bekannt. Geeignete Lösungsmittel sind beispielsweise Wasser, Acetonitril, DMSO, DMF, Dioxan, THF, Methanol oder Mischungen zweier oder mehrerer der genannten Lösungsmittel.

### 2. Festphasen-Synthese

Insbesondere, wenn das Konjugat ein oder mehrere Monomereinheiten enthalten soll, die nicht aus ε-Lysin bestehen oder die aus derivatisierten ε-Lysin-Monomereinheiten bestehen, kann eine Festphasensynthese zur Herstellung der erfindungsgemäßen Konjugate vorteilhaft sein. Diese Festphasensynthese wird entsprechend einer herkömmlichen Peptid-Synthese durchgeführt (z.B. Fmoc-Peptid-Synthese oder Boc-Peptid-Synthese). Dem Fachmann sind derartige Festphasensynthesen bekannt. Geeignete Lehrbücher für die Peptidsynthese sind Solid-Phase Peptide Synthesis: 289 (Methods in Enzymology) von Sidney P. Colowick (Autor), Gregg B. Fields (Herausgeber), Melvin I. Simon (Herausgeber) Academic Press Inc (November 1997) oder
Fmoc Solid Phase Peptide Synthesis: A Practical Approach von W. Chan (Autor), W. C. Chan (Herausgeber), Peter D. White (Herausgeber) "Oxford Univ Pr (2. März 2000). Die jeweils eingesetzten Monomere werden dabei so gewählt, dass ein Oligomer oder ein Konjugat entsprechend der vorliegenden Erfindung entsteht. Je nach Art der Monomereinheit kann zur Synthese direkt eine derivatisierte Monomereinheit verwendet werden oder eine zunächst an der für die Derivatisierung vorgesehenen Stelle geschützte Monomereinheit. Nach erfolgter Synthese des Oligomers kann dann entweder noch an der Festphase oder nach Abspaltung von der Festphase in Lösung die abschließende Derivatisierung mit den Carboxylgruppen tragenden Verbindungen, Wirkstoffen etc. vorgenommen werden.

Die Anbindung der Carboxylgruppen tragenden Verbindungen erfolgt in diesem Fall bevorzugt an das fertige Oligomer, d.h. entweder nach erfolgter Festphasensynthese des Oligomers noch an der Festphase oder nach dessen Abspaltung in Lösung.

Soll die Carboxylgruppen tragenden Verbindung oder ein Wirkstoff oder ähnliche Komponenten (im Folgenden wird das Verfahren beispielhaft für einen Komplexbildner beschrieben) beispielsweise an das N-terminale Ende des Oligomers gebunden werden, werden die Oligomere typischerweise mit einer Amino-terminalen Schutzgruppe erzeugt, wie etwa Fmoc. Kann der Komplexbildner die Bedingungen überstehen, welche verwendet werden, um das Oligomer von dem Syntheseharz abzuspalten und zum Entschützen der Seitenketten, kann das Fmoc von dem N-Terminus des vollständigen Harz-gebundenen Polypeptids abgespalten werden, so dass der Komplexbildner an das freie N-terminale Amin gebunden werden kann. In solchen Fällen wird der Komplexbildner typischerweise durch Verfahren aktiviert, die in der Technik allgemein dafür bekannt sind, dass sie eine aktive Ester- oder aktive Carbonatgruppe erzeugen, die wirkungsvoll ist, um eine Amid- bzw. Carbamatbindung mit der Oligomer-Aminogruppe zu bilden. Natürlich kann auch eine andere Verknüpfungschemie verwendet werden.

Um dabei zu helfen Nebenreaktionen zu minimieren, können Guanidino-und Amidinogruppen blockiert werden unter Verwendung herkömmlicher Schutzgruppen, wie etwa Carbobenzyloxygruppen (CBZ), di-*t*-BOC, PMC, Pbf, N-NO₂ und ähnlichen.

Kopplungsreaktionen werden durchgeführt durch bekannte Kopplungsverfahren in Lösungsmitteln, wie etwa N,N-Dimethylformamid (DMF), *N*-Methylpyrrolidon, Dichlormethan und/oder Wasser. Beispielhafte Kopplungsreagenzien umfassen O-Benzotriazolyloxytetramethyluroniumhexafluorphosphat (HATU), Dicyclohexylcarbodiimid, Brom-tris(pyrrolidino)phosphoniumbromid (PyBroP), usw. Andere Reagenzien können enthalten sein, wie etwa *N*,*N-*Dimethylaminopyridin (DMAP), 4-Pyrrolidinopyridin, *N*-Hydroxysuccinimid oder N-Hydroxybenzotriazol.

Entsprechend kann auch die Anbindung von Carboxylgruppen tragenden Verbindungen, Wirkstoffen, Komplexbildnern oder ähnliche Komponenten an die Aminogruppen der nicht endständigen ε-Lysin-Monomereinheiten erfolgen.

Enthält das Molekül Komplexbildner können die Metallionen nach bekannten Methoden komplexiert werden.

Die vorliegende Erfindung betrifft zudem die Verwendung der erfindungsgemäßen Konjugate zur Herstellung einer pharmazeutischen Zusammensetzung, insbesondere einer therapeutischen Zusammensetzung, oder einer bildverstärkenden Zusammensetzung (z.B. ein Kontrastmittel) oder eines radiomarkierten Tracers für die nuklearmedizinische Bildgebung..

Eine therapeutische Zusammensetzung besteht in der Regel zumindest aus dem Wirkstoff - in diesem Fall dem erfindungsgemäßen Konjugat mit angebundenem Wirkstoff - und einem geeigneten Lösungsmittel oder Trägerstoff, der Applikation der therapeutischen Zusammensetzung erlaubt. Für diagnostische Anwendungen dient das erfindungsgemäße Konjugat bevorzugt als Signalquelle in einem bildverstärkenden Kontrastmedium, so dass dieses mittels nuklearmedizinischer und/oder radiologischer Verfahren wie SPECT, PET, Ultraschall und oder auch durch Magnetresonanztomographie, computertomographische und optische Bildgebungsverfahren (Near-Infrared-Imaging) detektiert werden kann. Dem Fachmann sind Detektionsverfahren und Anwendungen für bildverstärkende Kontrastmedien bekannt. Beispiele für geeignete Anwendungen sind die Diagnostik von Krebserkrankungen, neurologische Fragestellungen, Überprüfung des Ansprechens auf eine Therapie, Überprüfung des Grads der Schädigung einer Niere bei z.B. Autoimmunerkrankungen und die Kontrolle von Gentherapien, jedoch auch die Erkennung von zellulären Veränderungen.

Bildverstärkende Zusammensetzungen bestehen in der Regel aus der Signalquelle, auch Diagnostikum genannt, und einem geeigneten Lösungsmittel oder Trägerstoff, der die Applikation des bildverstärkenden Kontrastmediums erlaubt.

Gegenstand der Erfindung sind ferner Arzneimittel bzw. pharmazeutische Zusammensetzungen, enthaltend mindestens ein erfindungsgemäßes Konjugat, dessen pharmazeutisch verwendbaren Salze und Stereoisomere, einschließlich deren Mischungen in allen Verhältnissen, sowie gegebenenfalls Träger- und/oder Hilfsstoffe.

Pharmazeutische Zusammensetzungen bzw. Arzneimittel lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren herge-stellt werden, indem beispielsweise der Wirkstoff mit dem bzw. den Trägerstoff(en) oder Hilfsstoff(en) zusammengebracht wird.

An die orale Verabreichung angepasste pharmazeutische Formulierungen können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nichttoxische und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Calciumstearat oder Polyethylenglykol in Festform können dem Pulvergemisch vor dem Füllvorgang zugesetzt werden. Ein Sprengmittel oder Lösungsvermittler, wie z.B. Agar-Agar, Calciumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder β-Lactose, Süßstoffe aus Mais, natürliche und synthetische Gummi, wie z.B. Akazia, Traganth oder Natriumalginat, Carboxymethylcellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylcellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylcellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dicalciumphosphat, vermischt wird. Das Pulvergemisch lässt sich granulieren, indem es mit einem Bindemittel, wie z.B. Sirup, Stärkepaste, Acadia-Schleim oder Lösungen aus Cellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die erfindungsgemäßen Konjugate lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Konjugate können auch unter Verwendung monoklonaler Antikörper als individuelle Träger, an die die Konjugate gekoppelt werden, zugeführt werden. Die Konjugate können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Poly-ε-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepasste pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepasste pharmazeutische Verbindungen können als Salben, Cremes, Suspensionen, Lotionen, Pulver, Lösungen, Pasten, Gele, Sprays, Aerosole oder Öle formuliert sein.

An die rektale Verabreichung angepasste pharmazeutische Formulierungen können in Form von Zäpfchen oder Einläufen dargereicht werden.

An die nasale Verabreichung angepasste pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

Zu den an die parenterale Verabreichung angepassten pharmazeutischen Formulierungen gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Bevorzugt werden die erfindungsgemäßen Konjugate parenteral verabreicht.

Es versteht sich, dass die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge des erfindungsgemäßen Konjugates hängt von einer Reihe von Faktoren ab, einschließlich Art des gekoppelten Wirkstoffs, dem Alter und Gewicht des Patienten, dem exakten Krankheitszustand, der der Behandlung bedarf, sowie seines Schweregrads, der Beschaffenheit der Formulierung sowie dem Verabreichungsweg.

Gegenstand der vorliegenden Erfindung ist auch ein Kit zur Herstellung einer pharmazeutischen Zusammensetzung, insbesondere einer bildverstärkenden oder therapeutischen Zusammensetzung, zumindest enthaltend ein erfindungsgemäßes Konjugat. Weist dieses Konjugat einen Komplexbildner auf, hat dieser bevorzugt noch keine Metallionen mit bildverstärkender oder therapeutischer Wirkung komplexiert. Das erfindungsgemäße Konjugat kann in dem Kit in einem Lösungsmittel (z.B. einem wässrigen Puffer) gelöst vorliegen oder bevorzugt als Lyophillisat.

Da die Metallionen, die vom Komplexbildner des erfindungsgemäßen Konjugats komplexiert werden, für viele Anwendungen radioaktiv sind, können pharmazeutische Zusammensetzungen, die das Konjugat enthalten, nicht beliebig lange im voraus hergestellt werden. Weiterhin sind aufgrund der Radioaktivität bei der Herstellung bestimmte Vorschriften zur Arbeitssicherheit zu beachten. Aus diesem Grund ist es erfindungsgemäß bevorzugt, einen Kit zur Verfügung zu stellen, der das erfindungsgemäße Konjugat enthält, wobei der Komplexbildner noch nicht die für die endgültige Anwendung notwendigen Metallionen komplexiert hat.

Es wurde gefunden, dass die erfindungsgemäßen Konjugate bereits kurze Zeit nach der Applikation ausschließlich, bzw. fast ausschließlich in der Niere angereichert werden. Im Falle der bevorzugten intravenösen Gabe der erfindungsgemäßen Konjugate ist schon nach 5 Minuten eine Anreicherung in der Niere zu beobachten. Nach einer Stunde befinden sich mehr als 50%, bevorzugt mehr als 70%, besonders bevorzugt mehr als 80%, ganz besonders bevorzugt mehr als 90% der injizierten Dosis in der Niere (%-Angaben basieren auf Messung der Radioaktivität). Dies ist insbesondere überraschend, weil entsprechende Versuche mit Konjugaten des α-Polylysins keine spezifische Anreicherung in der Niere zeigen.

Bei Organverteilungsstudien mit radiomarkierten erfindungsgemäßen Konjugaten (beispielsweise PET-Messungen oder nicht-invasiver Bildgebung) zeigen die erfindungsgemäßen Konjugate eine Stunde nach intravenöser Applikation typischerweise mindestens ein 10-fache, bevorzugt mindestens eine 20-fache Anreicherung in der Niere im Verhältnis zum übrigen Körper (Blut, Herz, Lunge, Milz, Leber, Muskel, Gehirn).

Daher können die erfindungsgemäßen Konjugate hervorragend für diagnostische Anwendungen, wie Nierenszintigraphie, Nieren-PET und Nieren-MRT, Funktionsprüfungen der Niere allgemein, zur Therapie und Diagnostik von Nierenkrebs und gegebenenfalls Metastasen von Nierenkrebs, CT der Niere und/oder Ultraschall der Niere eingesetzt werden.

Die therapeutische Anwendung liegt insbesondere im Drug Targeting für das Organ Niere. Insbesondere können an die erfindungsgemäßen Konjugate Wirkstoffe wie Antibiotika, Entzündungshemmer, ACE-Hemmer, Diuretika, Immunsuppressiva oder Chemotherapeutika, beispielsweise über spaltbare Spacer-Sequenzen, angebunden werden.

Auch der Einsatz der erfindungsgemäßen Konjugate zur Blockade der Rückresorption nierentoxischer Substanzen ist möglich.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen ist durch Bezugnahme in diese Anmeldung eingeführt.

### Beispiele

### 1. Materialsynthesen

### Beispiel 1 (ε-L-Polylysin-DOTA)

**DOTA-2,6-difluorphenylester:** Aus DOTA und 2,6-Difluorphenol mit DCC (Mier et al. Bioconjugate Chem. 2005, 16, 237)

ε-L-Polylysin, mittlere Molmasse ca. 4000 (hauptsächlich bestehend aus 29-34 Lysineinheiten) wird als 25% wässrige Lösung von der Firma Chisso Corp. (Japan) bezogen und lyophilisiert. ε-Polylysin (30 mg) wird in Wasser (200 µl) gelöst und mit einer Lösung von DOTA-2,6-difluorphenylester (100 mg) in Methanol (1 ml) versetzt und 100 µl *N*,*N*-Diisopropylethylamin hinzugefügt und 2 Tage bei RT gerührt. Dann wurde nochmals DOTA-2,6-difluorphenylester (100 mg) zugegeben und ü. N. bei RT gerührt. Dann wird mit Wasser verdünnt und präparativ mit HPLC aufgereinigt. Saubere Fraktionen werden zusammen lyophilisiert. DOTA-ε-Polylysin (98 mg) wird als farblose Festsubstanz erhalten. Die Anzahl der DOTA-Einheiten pro Molekül ε-Polylysin wird durch Beladung mit Gd und mit MS zu ca. 10 DOTA-Einheiten pro Molekül ε-Polylysin bestimmt; d. h. ca. 30% der Aminogruppen des ε-Polylysin haben reagiert.

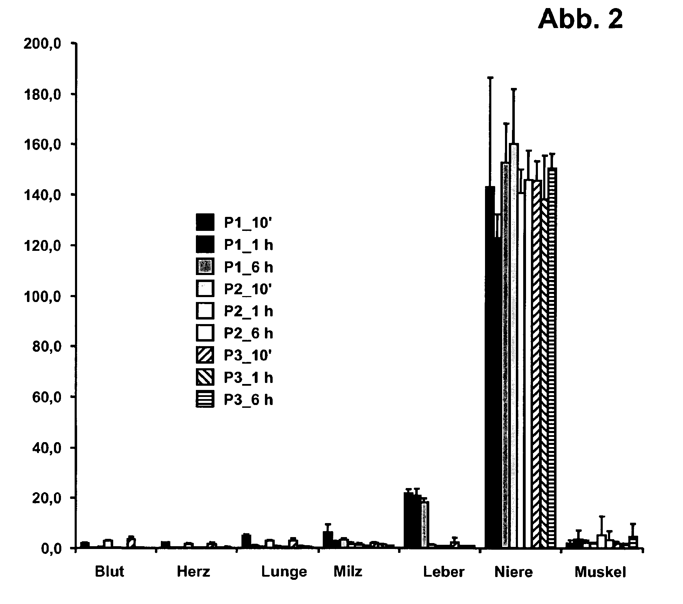

Eine schematische Darstellung der in Beispiel 1 erhaltenen Verbindung findet sich in Abbildung 2, wobei die DOTA-Modifikationen entgegen der vereinfachten Darstellung in Abbildung 2 in dem Molekül natürlich statistisch verteilt sind.

### Beispiel 2 (ε-L-Polylysin-DTPA)

75 mg ε-L-Polylysin und 310 mg DTPA-Difluorphenylester-tetra-*t*-butylester werden in 4 ml Methanol gelöst und 20 h bei RT gerührt. Die Reaktionslösung wird eingeengt und 4 ml TFA +100µl Wasser zum Rückstand gegeben und 20 h stehengelassen. Das Produkt wird mit Diethylether ausgefällt. Aufreinigung mit HPLC und Lyophilisieren ergibt 150 mg als farbloser Feststoff.

### Beispiel 3 (Beladung mit ¹¹¹In)

1.5 mg ε-L-Polylysin-DOTA werden in 500 µl Ethanol, 100 µl DMSO und 500 µl 0.4 M Natriumacetatpuffer, pH 5, gelöst. 30 µl von dieser Lösung werden mit 30 µl Puffer verdünnt, ¹¹¹InCl₃ (20 MBq) zugegeben und 8 min bei 70°C erhitzt. Radio-HPLC zeigt vollständige Komplexierung.

1.2 mg ε-L-Polylysin-DTPA werden in 400 µl 0.4 M Natriumacetatpuffer, pH 5, gelöst. 80 µl dieser Lösung werden mit 20 MBq ¹¹¹InCl₃ versetzt und 10 min auf 70°C erhitzt. Radio-HPLC zeigt vollständige Komplexierung.

### Beispiel 4 (Beladung mit ⁶⁸Ga)

50 µl der Lösung von ε-L-Polylysin-DOTA aus Beispiel 3 werden zu 100 µl einer Lösung von 50 MBq ⁶⁸GaCl₃ (pH 3) gegeben und 10 min bei 95°C erhitzt. HPLC zeigt vollständige Komplexierung.

### Beispiel 5 (Beladung mit Gd)

10 mg ε-L-Polylysin-DOTA und 50 mg GdCl₃-Hexahydrat werden in 600 µl 0.4 M Natriumacetatpuffer pH 5 30 min bei 60°C erhitzt. Aufreinigung mit HPLC und Lyophilisieren ergibt 10 mg als farbloser Feststoff.

### Beispiel 6 (EPL-DOTA-Modifizierung mit ¹⁹F)

50 mg ε-L-Polylysin-DOTA werden in 3 ml Methanol gelöst und mit 200 µl Triethylamin und 200 µl Trifluoressigsäureethylester versetzt und 1 h bei RT gerührt. Aufreinigung mit RP-HPLC unter Verwendung eines Elutionsgradienten aus Wasser und Acetonitril und Lyophilisieren ergibt 35 mg als farbloser Feststoff.

### Beispiel 7 (Enalaprilderivat von ε-Polylysin)

**Enalapril:** Enalapril-Maleat (Bosche Scientific) wird auf eine Säule mit Dowex 50WX8 gegeben und mit Wasser bis zur neutraler Reaktion gewaschen. Dann wird Enalapril mit 2% Pyridin in Wasser eluiert, Lösungsmittel werden eingeengt und Rückstand mehrmals mit Acetonitril eingeengt. Enalapril wird als farbloses Öl erhalten und direkt weiter verwendet.

**Enalapril-*t*-butylester:** Synthese erfolgt aus Enalapril und *N,N*'-Dicyclohexyl-O-t-butylisoharnstoffa (Chadran, Ravi US 2006241017)

**Enalaprilat-*t*-butylester:** Synthese erfolgt aus Enalapril-t-butylester mit NaOH (Iwasaki et al. Chem. Pharm. Bull. 37(2) 280 (1989)

***N,N*'-Diisopropyl-O-benzylisoharnstoff:** Synthese erfolgt aus Benzylalkohol und *N,N*'-Dicyclohexylcarbodiimid (Mathias, Synthesis **1979,** 561.)

**6-Hydroxy-hexansäurebenzylester:** Zu 6-Hydroxy-hexansäure (ABCR) (1,32 g; 10 mmol) wird eine Lösung von *N,N*'-diisopropyl-*O-*benzylisoharnstoff (2.34 g; 10 mmol) in THF (8 ml) gegeben und 2 Tage bei RT gerührt. Festes wird abfiltriert und Filtrat eingeengt. Rückstand wird an Kieselgel mit Ethylacetat:Hexan 1:2 zu 1:1 eluiert. Ausbeute 90%.

**Hexansäurebenzylester-6-*O*-*N,N*'-diisopropylisourea:** 6-Hydroxy-hexansäurebenzylester (2,0 g; 9 mmol) wird mit *N,N*'-Diisopropylcarbodiimid (1,14 g, 9 mmol) versetzt, CuCl (30 mg) und THF (6 ml) zugegeben und über Nacht bei RT gerührt. Dann wird mit THF verdünnt und in einer Fritte über neutralem Aluminiumoxid filtriert und mit THF nachgewaschen. Das Filtrat wird eingeengt, Rückstand in Diethylether aufgenommen, Festes abfiltriert und Filtrat eingeengt. Das Produkt wird ohne weitere Aufreinigung verwendet.

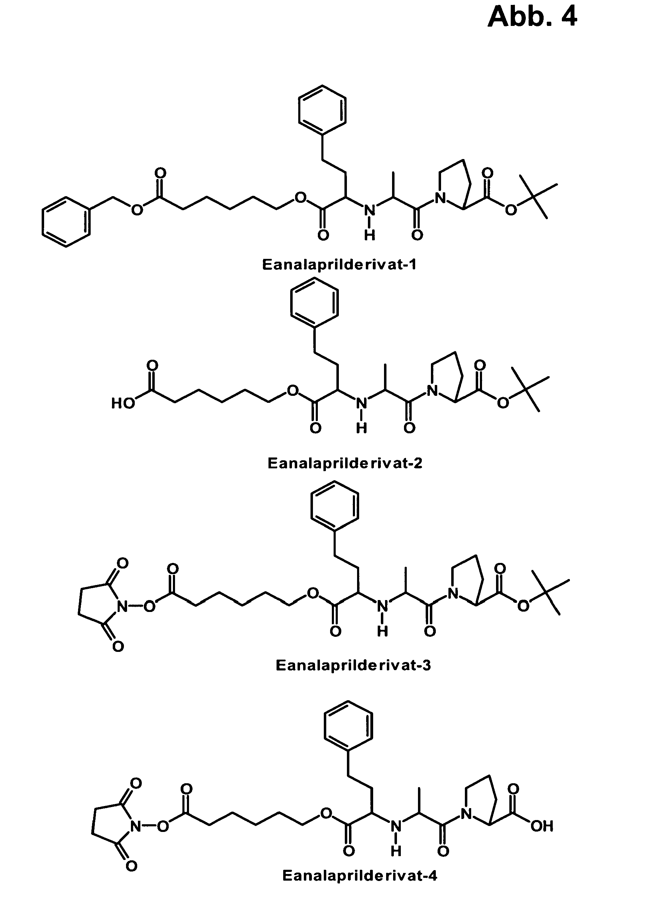

**Enalaprilderivat-1 (entsprechend** **Abb. 4****):** Zu Hexansäurebenzylester-6-*O*-*N,N*'-diisopropylisoharnstoff (2,47 g, 7,09 mmol) wird eine Lösung von Enalapril-t-butylester (2,87 g 7,09 mmol) in THF (20 ml) gegeben, 20 mg DMAP hinzugefügt und 7 h unter Rückfluß erhitzt, dann über Nacht bei 60°C gerührt. Die Reaktionslösung wird in Eis gekühlt und festes abfiltriert. Das Filtrat wird eingeengt und der Rückstand an Kieselgel mit Ethylacetat:Hexan 1:2 zu 2:1 eluiert. Saubere Fraktionen werden zusammen eingeengt. Ausbeute 85%.
¹³C und ¹H-NMR bestätigen die angegebene Struktur.

**Enalaprilderivat-2 (entsprechend** **Abb. 4****):** Enalaprilderivat-1 (400 mg) wird in Methanol (50 ml) gelöst, Pd/C 10% (70 mg) zugegeben und bei Normaldruck mit Wasserstoff hydriert. Nach 40 min zeigt HPLC vollständige Reaktion. Katalysator wird abfiltriert und Lösungsmittel eingeengt. Das Produkt wird ohne weitere Aufreinigung verwendet. ¹³C und ¹H-NMR bestätigen die angegebene Struktur.

**Enalaprilderivat-3 (entsprechend** **Abb. 4****):** Enalaprilderivat-2 (183 mg; 0,35 mmol) wird in Acetonitril (2 ml) gelöst und mit *N,N,N',N*'-Tetramethyl-*O*-(*N*-succimidyl)-uronium-tetrafluoroborat (TSTU) (106 mg; 0,35 mmol) in Acetonitril (2 ml) versetzt und *N,N*-Diisopropylethylamin (63 µl; 0,35 mmol) hinzugefügt und bei RT gerührt. HPLC zeigt nach 5 min vollständige Reaktion. Das Produkt wird präparativ HPLC aufgereinigt. Nach Lyophilisieren wird das Produkt als farblose halbfeste Verbindung erhalten. MS (Massenspektrometrie) zeigt die erwartete exakte Masse.

**Enalaprilderivat-4 (entsprechend** **Abb. 4****):** Enalaprilderivat-3 (8 mg) wird in einem Gemisch von TFA (200 µl) und Triisopropylsilan (5 µl) gelöst bei RT stehengelassen. HPLC zeigt nach 1 Std. vollständige Reaktion. Acetonitril (1 ml) wird zugegeben und dann eingeengt. Rückstand wird in 1 ml Acetonitril:Wasser 1:1 gelöst und lyophilisiert. MS zeigt die erwartete exakte Masse.

### ε-Polylysin-enalaprilderivat:

Lösung A: 8 mg Enalaprilderivat-4 in 100 µl Acetonitril
Lösung B: 100 mg ε-Polylysin in 650 µl Wasser und 350 µl Acetonitril

Zu 100 µl Lösung B wird 5 µl N,N-Diisopropylethylamin zugegeben und gemischt. Dann wird 25 µl Lösung A zugegeben und möglichst schnell gemischt. HPLC zeigt nach wenigen Minuten ein Peak von nichtabreagiertes ε-Polylysin und ein neues Hauptpeak aber kein Peak mehr für Enalaprilderivat-4. Durch präparative HPLC wird das neuentstandene Produkt isoliert und durch MS als das gewünschte mono-Enalapril-ε-polylysinderivat charakterisiert.

**DOTA-**ε**-polylysin-enalaprilderivat:** Zu ε-Polylysin-enalaprilderivat in Wasser wird Überschuss an DOTA-2,6-difluorphenylester in Methanol gegeben N,N-diisopropylethylamin hinzugefügt. Aufreinigung mit präparative HPLC ergibt die gewünschte Verbindung.

### 2. Anwendungsbeispiele

### 1. Organverteilungsstudie

Zur Ermittlung der Pharmakokinetik wird das ε-Polylysin-DOTA entsprechend Synthese-Beispiel 2/3 mit dem radioaktiven Nuklid ¹¹¹In markiert und NMRI-Mäusen in Gruppen zu jeweils drei Tieren über die Schwanzvene injiziert. Die verwendete Menge beträgt ca. 5 µg ε-Polylysin-DOTA pro Tier, die Dosis liegt bei ca. 1 MBq pro Tier.
Anschließend werden die Tiere seziert und die isolierten Organe (Blut, Herz, Lunge, Milz, Leber, Niere, Muskel, Gehirn) bezüglich ihrer Radioaktivität hin im Gamma-Counter untersucht. Die Menge an Radioaktivität im entsprechenden Organ repräsentiert direkt die aufgenommene Menge an ε-Polylysin-DOTA. Die Tiere werden nach verschiedenen Zeiten getötet. Dies erfolgt, wie die gesamte Versuchsdurchführung im Rahmen der ethischen Grundsätze für die wissenschaftliche Durchführung von Tierversuchen.
Überraschenderweise wird bei den Messungen festgestellt, dass sich bereits 10 Minuten nach der Injektion des mit ¹¹¹In beladenen ε-Polylysin-DOTA, über 150 Prozent der injizierten Dosis pro Gramm Nierengewebe in den Nieren der Versuchstiere anreichert. Ein kleiner Anteil wird über den Urin ausgeschieden.

Die graphische Darstellung der Anreicherung in den verschiedenen untersuchten Organen findet sich in Abbildung 2. Die Y-Achse stellt die spezifische Anreicherung in den jeweiligen Organen dar - angeben als Prozent der injizierten Dosis pro Gramm Gewebe. P1 bis P 3 stellen die Ergebnisse für Konjugate mit verschiedenem Beladungsgrad dar: P1: ca. 10% der Lysinmonomere tragen ein DOTA, P2: ca. 30% der Lysinmonomere tragen ein DOTA und P3: ca. 50% der Lysinmonomere tragen ein DOTA.

### 2. PET-Messung

Für PET-Messungen wird, entsprechend Synthese-Beispiel 4, mit 68-Gallium markiert und in einem Kleintier-PET-Scanner(Fa. Siemens) dynamisch die Nierenanreicherung untersucht. Hier zeigt sich, dass die Verbindung (ε-L-Polylysin-DOTA, beladen mit ⁶⁸Ga) ein außergewöhnlich gutes Organ- zu Hintergrund-Verhältnis erzielt und sich nahezu ausschließlich in der Niere anreichert. Typische Werte liegen dabei bei einer 20-fachen Anreicherung in der Niere im Verhältnis zum restlichen Körper.

### 3. Vergleichsversuche

In planaren Szintigraphien werden im Ratten- und im Mausmodell die Aufnahme von ^{99m}Tc-MAG3 und von ^{99m}Tc-DMSA verglichen mit der Aufnahme von (ε-L-Polylysin-DOTA, beladen mit ¹¹¹In). Beide bekannten Tracer zeigen ein schlechteres Nieren- zu Hintergrund-Verhältnis als die erfindungsgemäße Substanz.

## Patentansprüche

1. Konjugat enthaltend zumindest eine Carboxylgruppen tragende Verbindung und ein Oligomer, das aus peptidisch verknüpften Monomereinheiten besteht und das entweder zu mehr als 50% (bezogen auf die Anzahl der Monomereinheiten) aus ε-Lysin-Monomereinheiten aufgebaut ist oder mindestens 10 aufeinander folgende Monomereinheiten enthält, die zu mindestens zu 70% (bezogen auf die Anzahl der Monomereinheiten) aus ε-Lysin-Monomereinheiten aufgebaut sind.

2. Konjugat nach Anspruch 1, **dadurch gekennzeichnet, dass** das Konjugat zusätzlich zumindest einen Wirkstoff enthält.

3. Konjugat nach Anspruch 2, **dadurch gekennzeichnet, dass** der Wirkstoff ausgewählt ist aus der Gruppe der **Immunsuppressiva** z. B. Azathioprin, Mycophenolat-Mofetil, Ciclosporin, Tacrolimus, Sirolimus, Fingolimod oder Triptolid, **Zytostatika** z.B. Bleomycin, Dactinomycin, Mitomycin, Daunorubicin, Doxorubicin, Epirubicin, Idarubicin, Mitoxantron, Amascrin, Doxofluridin, Cisplatin, Carboplatin, Oxaliplatin, Satraplatin, Camptothecin, Toptecan, Irinotecan, Amsacrin, Etoposid, Teniposid, Cyclophosphamid, Trofosfamid, Melphalan, Chlorambucil, Estramustin, Busulfan, Chlorambucil, Chlormethin, Treosulfan, Carmustin, Lomustin, Nimustin, Procarbazin, Streptozocin, Dacarbazin, Ifosfamid, Temozolomid, Thiotepa, Vinorelbin, Vincristin, Vinblastin, Vindesin, Paclitaxel, Docetaxel, Methotrexat, Pemetrexed, Raltitrexed, Fluorouracil, Capecitabin, Cytosinarabinosid, Gemcitabin, Tioguanin, Pentostatin, Mercaptopurin, Fludarabin, Caldribin, Hydroxycarbamid, Mitotan, Azacitidin, Cytarabin, Nelarabin, Bortezomib, Anagrelid insbesondere der Protein-Kinase-Inhibitoren wie z. B. Imatinib, Erlotinib, Sunitinib, Sorafenib, Dasatinib, Lapatinib oder Nilotinib, **Immuntherapeutika** z. B. Cetuximab, Alemtuzumab und Bevacizumab, **Antiphlogistika** z. B. Naproxen, Ibuprofen, Indometacin, Prednisolon, Prednison, Hydrocortison oder Budesonid, Antibiotika insbesondere der Penicilline wie z. B. Benzylpenicillin, Methicillin oder Amoxicillin, der Cephalosporine wie z. B. Cefuroxim, Cefotaxim, Cefadroxil oder Cefixim, der β-Lactamase-Inhibitoren wie z. B. Clavulansäure, Sulbactam oder Tazobactam, der Carbapeneme wie z. B. Imipenem oder Meropenem, der Monobactame wie z. B. Aztreonam, der Tetracycline wie z. B. Tetracyclin, Chlortetracyclin, Oxytetracyclin, Doxycyclin, Minocyclin oder Tigecyclin, der Makrolid-Antibiotika wie z. B. Erythromycin A, der Glykopeptid-Antibiotika wie z. B. Vancomycin, der Endiine wie z.B. Calicheamicin, **Virostatika** z. B. Aciclovir, Valaciclovir, Ganciclovir, Valganciclovir, Penciclovir, Famciclovir, Brivudin, Cidofovir, Foscarnet, Idoxuridin oder Tromantadin, **Antihypertensiva** insbesondere der **ACE-Hemmer** wie z. B. Benazepril, Captopril, Cilazapril, Enalapril, Fosinopril, Lisinopril, Perindopril, Quinapril, Ramipril, Trandolapril oder Zofenopril, der **Sartane** wie z. B. Losartan, Balsartan, Irbesartan, Candesartan, Eprosartan, Olmesartan oder Telmisartan, der **Renin-Inhibitoren** wie z. B. Aliskiren und der **Betablocker** wie z. B. Proproanolol, Pindolol, Sotalol, Bopindolol, Atenolol, Bisorpolol, Celiprolol, Esmolol, Metoprolol, Nebivolol, Oxprenolol, Carvedilol oder Labetalol, **Urikosurika** z. B. Probenecid oder Benzbromaron oder **Diuretika** z. B. Acetazolamid, Furosemid, Torasemid, Bumetanid, Piretanid, Azosemid, Etacrynsäure, Etozolin, Hydrochlorothiazid, Benzthiazid, Chlorothiazid, Chlorthalidon, Indapamid, Mefrusid, Metolazon, Clopamid, Xipamid, Hydroflumethiazid, Methyclothiazid, Polythiazid, Amilorid, Triameteren, Spironolacton, Canrenon, Eplerenon oder Spironolacton.

4. Konjugat nach einem oder mehreren der Ansprüche 1 bis 3, **dadurch gekennzeichnet, dass** das Oligomer eine Kettenlänge von 10 bis 50 Monomereinheiten aufweist.

5. Konjugat nach einem oder mehreren der Ansprüche 1 bis 4, **dadurch gekennzeichnet, dass** das Oligomer mindestens eine der folgenden Monomereinheiten enthält:
-NH-SP-CO-
wobei SP eine C1 bis C20 Alkylen-, Alkenylen- oder Alkinylen-Gruppe sein kann, bei der ein oder mehrfach nicht benachbarte Methylengruppen ersetzt sein können durch -O-, -S-, -S(O)-, -SO₂-, -SO₂O-, -C(O)-, -C(O)O-, -CH₂-, -CHR'-, -CR'₂-, -CR'=CH-, -CH-CR'-, -CH=CH-, -CR'=CR'-, -C≡C-, - N⁺R'₂-, -P(O)R'O-, -C(O)NR'-, -SO₂NR'-, -OP(O)R'O-, -P(O)(NR'₂)NR'-, - PR'₂=N- oder -P(O)R'-
mit R' = C₁- bis C₆-Alkyl, C₃- bis C₇-Cycloalkyl, unsubstituiertes oder substituiertes Phenyl.

6. Konjugat nach einem oder mehreren der Ansprüche 1 bis 4, **dadurch gekennzeichnet, das** Oligomer nur aus ε-Lysin-Monomereinheiten besteht.

7. Konjugat nach Anspruch 6, **dadurch gekennzeichnet, dass** das Oligomer aus ε-Polylysin besteht.

8. Konjugat nach einem oder mehreren der Ansprüche 1 bis 7, **dadurch gekennzeichnet, dass** zumindest eine an das Oligomer konjugierte Carboxylgruppen tragende Verbindung zwei oder mehr freie Carboxylgruppen aufweist.

9. Konjugat nach einem oder mehreren der Ansprüche 1 bis 8, **dadurch gekennzeichnet, dass** zumindest eine Carboxylgruppen tragende Verbindung direkt oder über einen Spacer an die Aminogruppe einer ε-Lysin-Monomereinheit gebunden ist.

10. Konjugat nach einem oder mehreren der Ansprüche 1 bis 9, **dadurch gekennzeichnet, dass** es sich bei zumindest einer Carboxylgruppen tragenden Verbindung um einen Komplexbildner handelt.

11. Verfahren zur Herstellung eines Konjugats entsprechend einem oder mehreren der Ansprüche 1 bis 10, das zumindest die folgenden Verfahrensschritte aufweist:
a) Bereitstellen eines Oligomers das aus peptidisch verknüpften Monomereinheiten besteht und das entweder zu mehr als 50% (bezogen auf die Anzahl der Monomereinheiten) aus ε-Lysin-Monomereinheiten aufgebaut ist oder mindestens 10 aufeinander folgende Monomereinheiten enthält, die zu mindestens zu 70% (bezogen auf die Anzahl der Monomereinheiten) aus ε-Lysin-Monomereinheiten aufgebaut sind
b) Konjugation mindestens einer optional aktivierten Carboxylgruppen tragenden Verbindung an das Oligomer aus Schritt a)

12. Verwendung eines Konjugats entsprechend einem oder mehreren der Ansprüche 1 bis 10 zur Herstellung einer pharmazeutischen Zusammensetzung, wie insbesondere einer therapeutischen Zusammensetzung oder einer bildverstärkenden Zusammensetzung.

13. Verwendung eines Konjugats entsprechend einem oder mehreren der Ansprüche 1 bis 10 zur Herstellung von Makromolekül-Konjugaten, wobei zwei oder mehrere erfindungsgemäße Konjugate an ein Makromolekül gebunden werden.

14. Pharmazeutische Zusammensetzung, insbesondere eine therapeutische oder eine bildverstärkende Zusammensetzung, zumindest enthaltend ein Konjugat entsprechend einem oder mehreren der Ansprüche 1 bis 10.

15. Kit zur Herstellung einer pharmazeutischen Zusammensetzung, insbesondere einer therapeutischen oder bildverstärkenden Zusammensetzung, zumindest enthaltend eine Konjugat entsprechend einem oder mehreren der Ansprüche 1 bis 10.
